(19) Europäisches Patentamt
European Patent Office
Office européen des brevets

(11) Publication number: **0 081 584**
**B1**

# (12) EUROPEAN PATENT SPECIFICATION

(45) Date of publication of patent specification: **12.02.86**

(51) Int. Cl.[4]: **C 07 D 513/04,** A 01 N 43/90

(21) Application number: **81901677.5**

(22) Date of filing: **15.06.81**

(86) International application number:
**PCT/JP81/00139**

(87) International publication number:
**WO 82/04436 23.12.82 Gazette 82/30**

## (54) THIAZOLINOTRIAZINE DERIVATIVES.

(43) Date of publication of application:
**22.06.83 Bulletin 83/25**

(45) Publication of the grant of the patent:
**12.02.86 Bulletin 86/07**

(84) Designated Contracting States:
**CH DE FR GB LI NL**

(56) References cited:
**DE-A-1 670 948**
**JP-A-53 084 992**
**JP-A-56 051 489**
**JP-B-47 012 350**
**JP-B-53 037 358**
**JP-B-54 029 519**

(73) Proprietor: **NIPPON SODA CO., LTD.**
**2-1, Ohte-machi 2-chome**
**Chiyoda-ku, Tokyo, 100 (JP)**

(72) Inventor: **MAEDA, Kuniyasu**
**13-4 Sakuragaoka Kukizaki-cho**
**Inashiki-gun Ibaragi-ken 300-12 (JP)**
Inventor: **KAERIYAMA, Minoru**
**279 Nakazato Odawara-shi**
**Kanagawa 250 (JP)**
Inventor: **MATSUI, Nobuo**
**198 Ohaza Hattomakishinden Nakagou-mura**
**Nakakubiki-gun Niigata-ken 949-23 (JP)**
Inventor: **MIZUNO, Masami**
**3-105 Higiriyama 1-chome Konan-ku**
**Yokohama-shi Kanagawa-ken 233 (JP)**
Inventor: **YASUDA, Yasushi**
**2308 Shimonagayacho Konan-ku**
**Yokohama-shi Kanagawa 233 (JP)**
Inventor: **NAKATA, Akira**
**11-15 Otowa-cho 6-chome Fujieda-shi**
**Shizuoka-ken 426 (JP)**

(74) Representative: **van der Beek, George Frans et al**
**Nederlandsch Octrooibureau Johan de Wittlaan**
**15 P.O. Box 29720**
**NL-2502 LS 's-Gravenhage (NL)**

**EP 0 081 584 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field:

This invention relates to novel thiazolinotriazine derivatives, to a process for the preparation thereof, and to its use as fungicides for agriculture and horticulture.

Background Art:

Many agricultural fungicides used for plant diseases have been developed, however, only a few of them are effective with no phytotoxicity against control of such diseases as phytophthora diseases and downy mildew of fruit trees and vegetable. And, "chlorothalonil" (common name) is recognized as one of typical commercial products meeting the requirements.

Disclosure of Invention:

Thiazolinotriazine derivatives having the formula

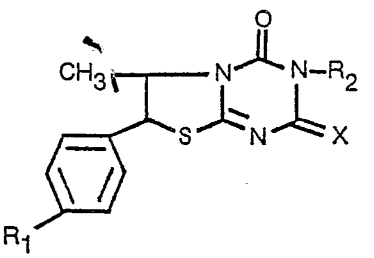

[I]

wherein $R_1$ denotes hydrogen atom, halogen group atom or methyl radical,

$R_2$ denotes cyclohexyl radical, phenyl radical or phenyl radical substituted with methyl radical or nitro radical, and

X denotes oxygen atom or sulfur atom, are novel compound.

The compounds of formula (I) are useful as agricultural and horticultural fungicides.

The compounds of formula (I) can be prepared by reacting compounds having the formula

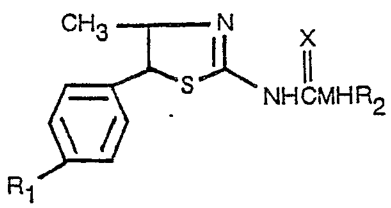

[II]

wherein $R_1$, $R_2$ and X are defined previously, are reacted with phosgene, trichloromethyl chloroformate, a chloroformic ester or carbonyldiimidazol in the presence of an acid-binding agent.

Best Mode of Carrying Out the Invention:

Among the compounds of formula (I), superior fungicidal effectiveness is generally obtained for compounds having cyclohexyl radical as $R_2$. Furthermore, though the compounds of formula (I) include optical isomers, cis-form isomers are usually exhibit the strongest fungicidal activity.

The compounds of formula (I) are especially useful for the control of phytophthora diseases and downy mildew of vegetable and fruit trees, and no phytotoxicity is observed when applied.

The preparation reactions are carried out in an inert organic solvent such as ethyl acetate, benzene and chloroform. The compound of formula (II) and an acid-binding agent such bases as dimethylaniline and triethylamine are dissolved in the organic solvent. A carbonic acid derivative selected from phosgene, trichloromethylchloroformate, chloroformate esters and carbodiimidazole is added to the solution, and the reaction is usually continued for 1 hour to several hours at temperatures from 0°C to boiling point of the organic solvent. After the reaction is completed, the reaction mixture is washed with water and the solvent is removed to obtain the product.

The compound of formula (II) may be prepared, for example, in accordance with the following reactions:

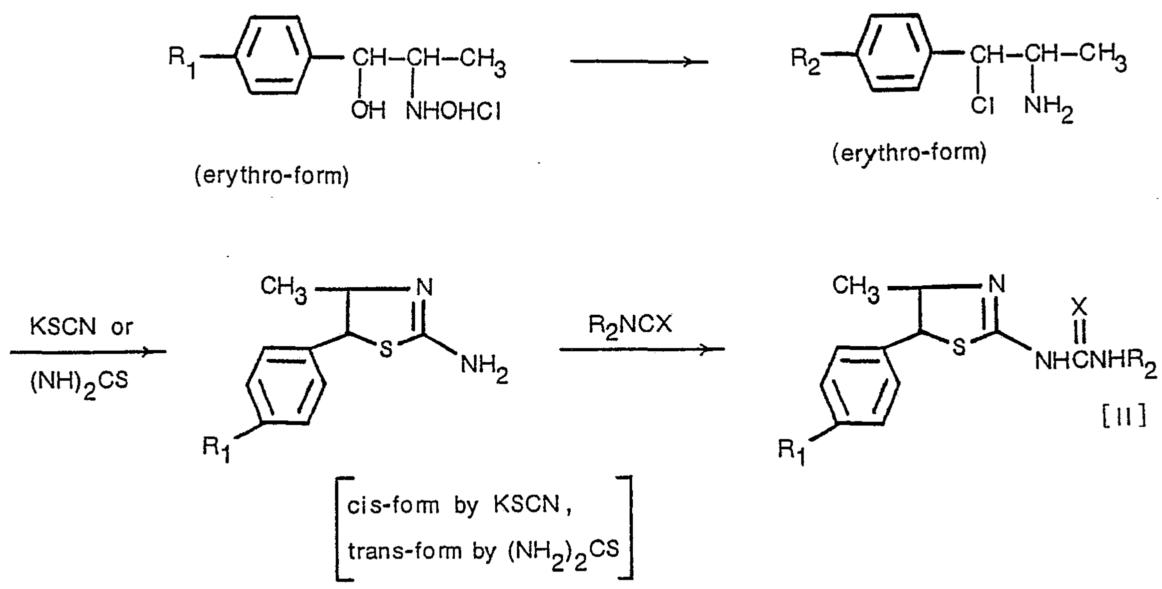


wherein R1, R2 and X are as defined previously.

Followings are non-limiting examples of preparing compounds of this invention.

Example 1

Preparation of 3-cyclohexyl-6-methyl-7-phenyl-3,4-dihydro-2H-thiazolino(3,2-a)-1,3,5-triazine-2,4-dione

3.4-gr of 1-(5-phenyl-4-methyl-$\Delta^2$-thiazoline-2-yl)-3-cyclohexylurea and 2.6 gr of N,N-dimethylaniline were dissolved in 70 ml of ethyl acetate and 10 ml of ethyl acetate which dissolved 1.3 gr of trichloromethylchloroformate was added dropwise in the solution at 5°C with stirring.

After 1 hour stirring, obtained reaction solution was washed with water and dried over anhydrous magunesium sulfate and solvent was distilled off. Obtained residue was recrystallized from ethanol and 2.8 gr of crystal having 168—169°C of melting point was obtained.

Example 2

Preparation of 3-cyclohexyl-6-methyl-7-(-4-methylphenyl)-3,4-dihydro-2H-thiazolino(3,2-a)-1,3,5-triazine-2,4-dione

0.9 gr of 1-(4-methyl-5-(4-methylphenyl)-$\Delta^2$-thiazolino-2-yl)-3-cyclohexy-lurea and 0.6 gr of N,N-dimethylaniline were dissolved in 30 ml of ethyl acetate and 10 ml of ethyl acetate which dissolved 0.4 gr of trichloromethylchloroformate was added dropwise in the solution at 5°C with stirring.

After 1 hour stirring, obtained reaction solution was washed with water and dried over anhydrous magunesium sulfate and solvent was distilled off. Obtained crystal was then washed with Ligroin and 0.45 gr of crystal having 162—165°C of melting point was obtained.

Example 3

Preparation of 3-cyclohexyl-6-methyl-7-phenyl-3,4-dihydro-2H-thiazolino(3,2-a)-1,3,5-triazine-2-thioxo-4-one

2.0 gr of 1-(5-phenyl-4-methyl-$\Delta^2$-thiazoline-2-yl)-3-cyclohexylthiourea and 1.45 gr of N,N-dimethylaniline were dissolved in 80 ml of ethyl acetate and 10 ml of ethyl acetate which dissolved 1.2 gr of trichloromethylchloroformate was added dropwise in the solution at 5°C with stirring.

After 30 minutes of stirring, the mixture was heated under reflux for 3 hours and cooled to room temperature. The reaction mixture was washed with water and dried over anhydrous magunesium sulfate, and solvent was distilled off. Obtained residue was recrystallized from methanol and 1.0 gr of crystal having 192—193°C of melting point was obtained.

Example 4

Preparation of trans-7-(p-chlorophenyl)-6-methyl-3-cyclohexyl-3,4-dihydro-2H-thiazolino(3,2-a)-1,3,5-triazine-2,4-dione

4 gr of 1-(trans-5-(p-chlorophenyl)-4-methyl-$\Delta^2$-thiazoline-2-yl)-3-cyclohexylurea and 3.3 gr of N,N-dimethylaniline were dissolved in 50 ml of ethyl acetate and then 1.25 gr of trichloromethylchloroformate was added dropwise. The mixture was kept stirring at 5°C for 30 minutes and further for 30 minutes at room temperature. Then water was added to from oil layer. Oil layer was isolated, washed with water and dried over anhydrous magunesium sulfate, and solvent was distilled off. Obtained crystal was washed with ligroin and 3.5 grs of crystal having 132—134°C of melting point was obtained.

Example 5

Preparation of trans-7-(p-chlorophenyl)-6-methyl-3-cyclohexyl-3,4-dihydro-2H-thiazolino(3,2-a)-1,3,5-triazine-2,4-dione

4 gr of 1-(trans-5-(p-chlorophenyl)-4-methyl-$\Delta^2$-thiazoline-2-yl)-3-cyclohexylurea and 3.3 gr of N,N-dimethylaniline were dissolved in 50 ml of chloroform and 1.25 gr of phosgene was introduced thereto at 5°C under stirring. The mixture was kept stirring at 5°C for 30 minutes and further for 30 minutes at room temperature. The mixture was treated as Example 4 and 3.8 gr of crystal having 132—134°C of melting point was obtained.

In table 1, typical compounds of this invention was listed.

TABLE 1

| No. | Structural Formula 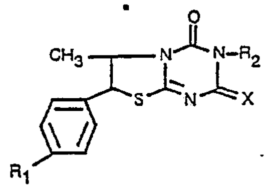 R1 | R2 | X | Stereoisomer | Melting Point [°C] or Refractive Index |
|---|---|---|---|---|---|
| 1 | H | 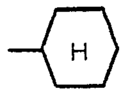 | O | cis | [168 – 169] |
| 2 | H | 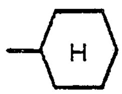 | S | cis | [192 – 193] |
| 3 | H | 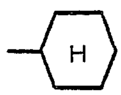 | O | trans | $n_D^{31}$ 1,5928 |
| 4 | $CH_3$ | 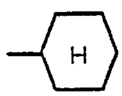 | O | cis | [162 – 165] |
| 5 | $CH_3$ | 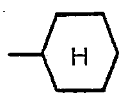 | O | trans | [136 – 138] |
| 6 | H | 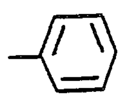 | O | cis | [183 – 184] |
| 7 | H | 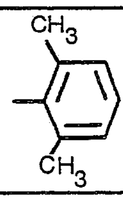 | O | cis | [180 – 182] |
| 8 | H | 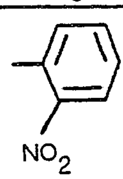 | O | cis | [225 – 227] |
| 9 | Cl | 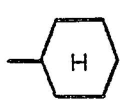 | O | trans | [132 – 134] |
| 10 | Cl | 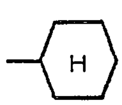 | O | cis | [205 – 207] |

The compounds according to this invention are utilized, if desired, in a form of the usual fungicide, and valious kinds of formulation, for example, wettable powder, emulsifiable concentrate and dust formulation can be applied. As additives or carriers, for solid type formulation, mineral powder such as diatom earth, apatite, talc, pyrophyllite and clay, vegetable powder such as soy-bean flour and wheat flour, for liquid type formulation, organic solvent such inert organic liquids as kerosene, mineral oil, petroleum, solvent naphtha, xylene, cyclohexane, cyclohexanone, dimethylformamide, alcohol and acetate, as well as water are employed. Conventional fungicidal surface-active agent may be used when homogeneous and stable formulations are desired.

Non-limiting examples of the fungicide of the invention are shown below, but additives and concentrations shall not be limited by these examples.

Example 6

Wettable Powder

| | |
|---|---|
| Compound 1 | 20 parts by weight |
| Diatom earth | 73 " |
| Sodium alkylarylsulfonate | 7 " |

These components were mixed and crushed finely and wettable powder containing 20% of active ingredient was obtained.

Example 7

Emulsible Concentrate

| | |
|---|---|
| Compound 2 | 20 parts by weight |
| Xylene | 42 " |
| Dimethylformamide | 30 " |
| Polyoxiethylenealkylarylether | 8 " |

These components were mixed and emulsible concentrate containing 20% of active ingredient was obtained.

Example 8

Dust Formulation

| | |
|---|---|
| Compound 4 | 2 parts by weight |
| Talc | 49 " |
| Clay | 49 " |

These components were mixed and crushed and dust formulation containing 2% of active ingredient was obtained.

Example 9

Granular Formulation

| | |
|---|---|
| Compound 7 | 5 parts by weight |
| Talc | 40 " |
| Clay | 39 " |
| Hentonite | 10 " |
| Sodium alkylsulfate | 6 " |

These components were mixed, finely crashed and then granulated to granular having particle size of 0.5—1.0 mm in diameter, and then granular containing 5% of active ingredient was obtained.

Obtained wettable powder and emulsifiable concentrate can be used by adding water in the forms of suspension or emulsion, and dust formulation and granular formulation can be used for prevention of plant diseases or control of plant diseases.

The fungicidal effects of compounds are illustrated by the following Tests:

Test 1 Test for control of Cucumber Downy Mildew

5 ml of an aqueous suspension containing desired amount of each compound was sprayed on a potted cucumber seedling (Variety: Satsukimidori) of 1.5 leaf stage and each treated cucumber was air-dried. Then Downy Mildew (*Pseudoperonospora cubensis*) was inoculated and had been kept in a greenhouse to cause the disease.

The occurence of Cucumber Downy Mildew and phyto-toxicity were inventigated, and effect for control of the disease was evaluated in comparison with occurrence of the disease on untreated cucumber leaves.

The results are shown in Table 2.

TABLE 2

| Compound No. | Concentration of active component (ppm) | Control effect (%) | Phytotoxicity |
|---|---|---|---|
| 1 | 400<br>200<br>100<br>50<br>25 | 100<br>100<br>100<br>100<br>100 | not observed |
| 2 | 400<br>200<br>100<br>50<br>25 | 100<br>100<br>100<br>100<br>100 | ditto |
| 3 | 400 | 100 | ditto |
| 4 | 400<br>200<br>100<br>50<br>25 | 100<br>100<br>100<br>100<br>100 | ditto |
| 5 | 400 | 100 | ditto |
| 6 | 400 | 100 | ditto |
| 7 | 400 | 100 | ditto |
| 8 | 400 | 100 | ditto |

TABLE 2 (Continued)

| Compound No. | Concentration of active component (ppm) | Control effect (%) | Phytotoxicity |
|---|---|---|---|
| 9 | 400 | 100 | ditto |
| | 200 | 100 | |
| | 100 | 100 | |
| | 50 | 100 | |
| | 25 | 100 | |
| 10 | 400 | 100 | not observed |
| | 200 | 100 | |
| | 100 | 100 | |
| | 50 | 100 | |
| | 25 | 100 | |
| tetrachloroiso phthalonitrile (chlorocholonic) | 400 | 90 | ditto |
| | 200 | 80 | |
| | 100 | 75 | |
| | 50 | 50 | |
| | 25 | 50 | |

## Claims

1. A compound having the formula

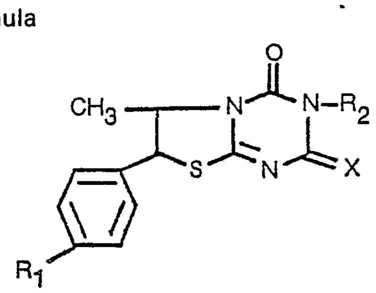

wherein $R_1$ denotes hydrogen atom, halogen group atom or methyl radical,

$R_2$ denotes cyclohexyl radical, phenyl radical or phenyl radical substituted with methyl radical or nitro radical, and

X denotes oxygen atom or sulfur atom.

2. A compound according to claim 1, wherein $R_1$ is chlorine atom, $R_2$ is cyclohexyl, and X is oxygen atom.

3. A process for the production of a compound according to claim 1 which comprises reacting a compound of the formula

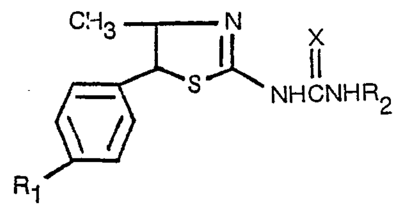

wherein $R_1$, $R_2$ and X are defined previously, with phosgene, trichloromethyl chloroformate, a chloroformic ester or carbonyldiimidazole in the presence of an acid-binding agent.

4. A fungicidal composition which comprises as an effective component a compound of the formula

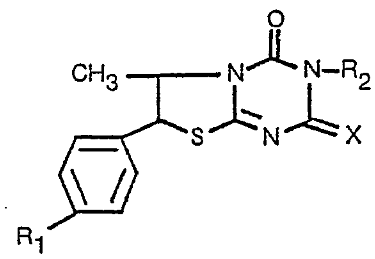

wherein $R_1$ denotes hydrogen atom, halogen group atom or methyl radical,

$R_2$ denotes cyclohexyl radical, phenyl radical or phenyl radical substituted with methyl radical or nitro radical, and

X denotes oxygen atom or sulfur atom.

**Patentansprüche**

1. Verbindung der Formel

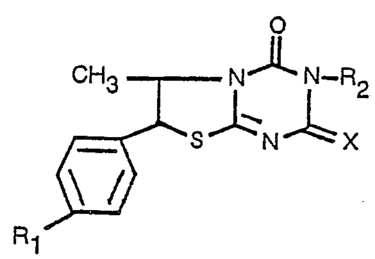

in der $R_1$ ein Wasserstoffatom, ein Atom aus der Gruppe der Halogene oder eine Methylgruppe bedeutet,

$R_2$ eine Cyclohexylgruppe, eine Phenylgruppe oder eine durch Methyl oder Nitro substituierte Phenylgruppe bedeutet und

X ein Sauerstoffatom oder ein Schwefelatom bedeutet.

2. Verbindung nach Anspruch 1, wobei $R_1$ ein Chloratom, $R_2$ eine Cyclohexylgruppe und X ein Sauerstoffatom ist.

3. Verfahren zur herstellung einer Verbindung nach Anspruch 1, umfassend die Umsetzung einer Verbindung der Formel

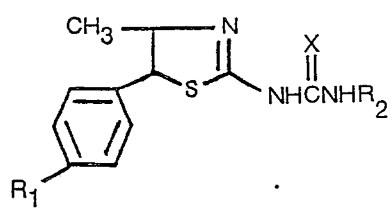

in der $R_1$, $R_2$ und X wie oben definiert sind mit Phosgen, Trichlormethylchlorformiat, einem Chlorameisensäureester oder Carbonyldiimidazol in Gegenwart eines säurebindenden Mittels.

4. Fungicides Mittel, enthaltend als wirksamen Bestandteil eine Verbindung der Formel

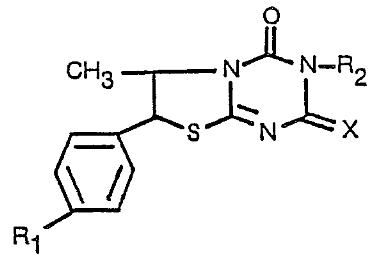

in der $R_1$ ein Wasserstoffatom, ein Atom aus der Gruppe der Halogene oder eine Methylgruppe bedeutet,

$R_2$ eine Cyclohexylgruppe, eine Phenylgruppe oder eine durch Methyl oder Nitro substituierte Phenylgruppe bedeutet und

X ein Sauerstoffatom oder ein Schwefelatom bedeutet.

**Revendications**

1. Composé ayant pour formule

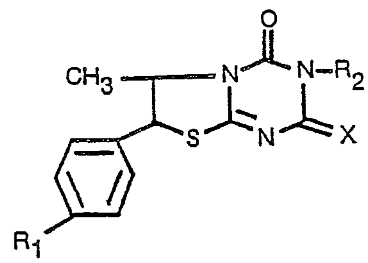

où $R_1$ désigne un atome d'hydrogéne, un atome du groupe halogène ou un radical méthyle,

$R_2$ désigne un radical cyclohexyle, un radical phényle ou un radical phényle substitué par un radical méthyle ou un radical nitro, et

X désigne un atome d'oxygène ou un atome de soufre.

2. Composé selon la revendication 1 où $R_1$ est un atome de chlore, $R_2$ est cyclohexyle, et X est un atome d'oxygène.

3. Procédé pour la production d'un composé selon la revendication 1 qui consiste à faire réagir un composé de formule

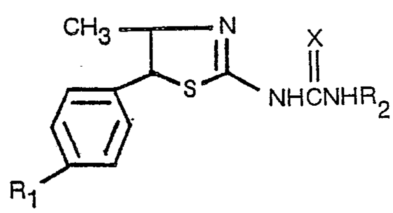

où $R_1$, $R_2$ et X sont précédemment définis, avec du phosgène, du trichlorométhyl chloroformiate, un ester chloroformique ou du carbonyldiimidazole en présence d'un agent liant l'acide.

4. Composition fongicide qui comprend comme composant efficace, un composé de formule

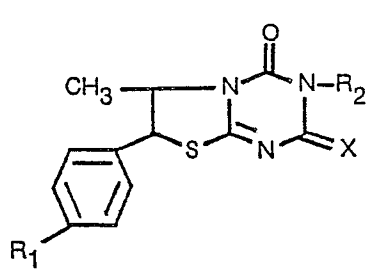

où $R_1$ désigne un atome d'hydrogène, un atome du groupe halogène ou un radical méthyle,

$R_2$ désigne un radical cyclohexyle, un radical phényle ou un radical phényle substitué par un radical méthyle ou un radical nitro et

X désigne un atome d'oxygène ou un atome de soufre.